# EUROPEAN PATENT SPECIFICATION

(11) **EP 3 218 512 B1**
(45) Date of publication and mention of the grant of the patent: **04.09.2019**
(21) Application number: 15797148.2
(22) Date of filing: 12.11.2015
(51) Int. Cl.: C12Q 1/68

(54) **A METHOD OF SCREENING FOR MODULATION OF CELL SIGNALLING PATHWAYS**
SCREENING-VERFAHREN ZUR MODULATION VON ZELLSIGNALWEGEN
PROCÉDÉ DE CRIBLAGE SUIVANT LA MODULATION DE VOIES DE SIGNALISATION CELLULAIRE

(30) Priority: 13.11.2014 GB 201420218
(43) Date of publication of application: 20.09.2017
(73) Proprietor: Cambridge Enterprise Limited, Cambridge CB2 1TN (GB)
(72) Inventor: HARDWICK, Bryn, Trinity Lane, Cambridge CB2 1TN (GB); VENKITARAMAN, Ashok, Trinity Lane, Cambridge CB2 1TN (GB); EMERY, Amy, Trinity Lane, Cambridge CB2 1TN (GB); MCKENZIE, Grahame, London WC2B 4AN (GB)
(74) Representative: Stratagem IPM Limited
(86) International application number: PCT/GB2015/053432
(87) International publication number: WO 2016/075468

(56) References cited:
- WO-A1-01/66787
- WO-A1-2009/127872
- WO-A1-2013/116903
- WO-A2-2012/012739
- US-A1- 2013 137 604
- US-B1- 6 890 716
- ROSARIO DEL M ET AL: "POSITIVE SELECTION SYSTEM TO SCREEN FOR INHIBITORS OF HUMAN IMMUNODEFICIENCY VIRUS-1 TRANSCRIPTION", BIOTECHNOLOGY. THE INTERNATIONAL MONTHLY FOR INDUSTRIAL BIOLOGY, NATURE PUBLISHING GROUP, US, vol. 14, no. 11, 1 January 1996 (1996-01-01), pages 1592-1596, XP000196943, ISSN: 0733-222X & Shaw-Yi Kao ET AL: "Anti-termination of transcription within the long terminal repeat of HIV-1 by tat gene product", NATURE, vol. 330, no. 6147, 3 December 1987 (1987-12-03), pages 489-493, XP055240443, United Kingdom ISSN: 0028-0836, DOI: 10.1038/330489a0
- RICHARD N. DAY ET AL: "The fluorescent protein palette: tools for cellular imaging", CHEMICAL SOCIETY REVIEWS., vol. 38, no. 10, 1 October 2009 (2009-10-01), page 2887, XP055242434, GB ISSN: 0306-0012, DOI: 10.1039/b901966a
- SO WON OH ET AL: "In vivo Monitoring of microRNA Biogenesis Using Reporter Gene Imaging", THERANOSTICS, vol. 3, no. 12, 8 December 2013 (2013-12-08), pages 1004-1011, XP055243179, Australia ISSN: 1838-7640, DOI: 10.7150/thno.4580
- C. M. CONNELLY ET AL: "High-Throughput Luciferase Reporter Assay for Small-Molecule Inhibitors of MicroRNA Function", JOURNAL OF BIOMOLECULAR SCREENING, vol. 17, no. 6, 12 March 2012 (2012-03-12) , pages 822-828, XP055241097, US ISSN: 1087-0571, DOI: 10.1177/1087057112439606
- KATO Y ET AL: "Real-time functional imaging for monitoring miR-133 during myogenic differentiation", INTERNATIONAL JOURNAL OF BIOCHEMISTRY AND CELL BIOLOGY, PERGAMON, GB, vol. 41, no. 11, 1 November 2009 (2009-11-01), pages 2225-2231, XP026644031, ISSN: 1357-2725, DOI: 10.1016/J.BIOCEL.2009.04.018 [retrieved on 2009-05-03]
- VICENT PELECHANO ET AL: "Gene regulation by antisense transcription", NATURE REVIEWS GENETICS, vol. 14, no. 12, 12 November 2013 (2013-11-12), pages 880-893, XP055243155, GB ISSN: 1471-0056, DOI: 10.1038/nrg3594
- ROSS C. WILSON ET AL: "Molecular Mechanisms of RNA Interference", ANNUAL REVIEW OF BIOPHYSICS, vol. 42, no. 1, 6 May 2013 (2013-05-06), pages 217-239, XP055243150, ISSN: 1936-122X, DOI: 10.1146/annurev-biophys-083012-130404

## Description

### Field of the Invention

This invention relates to methods for the identification of new therapeutic targets and protein interaction sites for use in drug discovery.

### Background of the Invention

The identification of new therapeutic targets is a key starting point for drug discovery. Drug discovery efforts have traditionally been focussed upon identifying classically-druggable targets such as kinases, G-protein coupled receptors (GPCRs) and ion channels. However, the ability to screen new classes of targets would allow the expansion of the 'druggable genome' and the identification of new therapeutic targets. Targets involved in protein:protein interactions (PPIs) are of particular interest, particularly PPIs which are involved in the signalling pathways which are utilised by cancer cells.

Functional screening and validation of candidate drug targets that are linked to disease biology has been performed typically at the genomic level, for example using gene knock-outs and the transcriptomic level (RNAi), where the number of targets is relatively limited (∼25,000 genes, with a subset of these having a number of alternative splicing forms). This level of complexity has not placed serious technical restrictions on assaying each gene-form sequentially for its function and essentiality in any disease setting of interest.

However, complexity at the proteomic level is substantially higher, with each protein adopting many different conformations during their normal or disease-context functions, which can offer numerous opportunities to therapeutically alter their activity. Because of this complexity, there have been few efforts to globally identify and functionally validate targets at the protein level, and the drug-binding sites therein. Although the concept has been proven using peptide libraries (<10,000 library size) in a 'well-by-well' basis to probe target function in phenotypic screening formats, this low-throughput approach is insufficient to routinely isolate new drug target sites for therapeutic intervention.

The current invention provides a method that allows the full complexity of the proteome (or genome) to be screened in pooled phenotypic assay formats, with a high degree of accuracy, enabling a clear and routine linkage between target and disease to be established. The invention further provides for the identification of protein interaction sites associated with disease.

### Summary of the Invention

This invention relates to the development of screening methods for peptides and nucleic acids which inhibit or block cell signalling pathways. These methods may be useful, for example, in identifying and characterising target proteins and also protein:protein interaction (PPI) sites for use as drug targets for the modulation of these pathways. One aspect of the invention provides a method of screening for a compound which inhibits a cell signalling pathway comprising:
(1) providing a population of cultured mammalian cells having an active cell signalling pathway, each mammalian cell comprising:
   (a) a first heterologous nucleic acid comprising;
      (i) a nucleotide sequence encoding a first detectable reporter and,
      (ii) a constitutive regulatory element which is operably linked to the nucleotide sequence; and,
   (b) a second heterologous nucleic acid comprising:
      (i) a nucleotide sequence encoding a repressor molecule which inhibits, inactivates or suppresses expression of the first detectable reporter,
      (ii) a nucleotide sequence encoding a second detectable reporter; and,
      (iii) a signal-activated regulatory element which is activated by said cell signalling pathway,
   said signal-activated regulatory element being operably linked to the repressor nucleotide sequence and the nucleotide sequence which encodes the second detectable reporter,
(2) introducing a library of test compounds into said population of mammalian cells, and;
(3) determining the expression of the first and the second detectable reporters in one or more of the population of transfected cells,
   wherein expression of the first detectable reporter but not the second detectable reporter in a transfected cell is indicative that the test compound inhibits said cell signalling pathway,
   wherein the expression of the first detectable reporter is fine-tuned by:
   (a) regulating the expression of the first detectable reporter using promoters of differing strengths; and/or
   (b) including an in-frame destabilisation nucleotide sequence within the nucleotide sequence of the first detectable reporter; and/or
   (c) including a nucleotide sequence which targets proteins for proteosomal degradation in the nucleotide sequence encoding a first detectable reporter; and/or
   (d) altering the stability of the first detectable reporter by incorporating adenylate-uridylate-rich elements (ARE's) into the 3' untranslated region (UTR) of its messenger RNA,
   optionally,
(4) identifying one or more cells in the population which express the first detectable reporter but not the second detectable reporter, wherein the test compound introduced into the one or more cells is a putative inhibitor of the cell signalling pathway,
preferably wherein the cell signalling pathway is constitutively activated in the mammalian cell,
preferably wherein the population of mammalian cells is provided by transfecting a population of mammalian cells with the first and second heterologous nucleic acids, preferably wherein the first and second heterologous nucleic acids are comprised within the same or separate expression vectors,
preferably wherein the repressor molecule is a miRNA.

The test compound may be a biomolecule which is introduced to the cell by contacting the cells with the biomolecule or by expressing a nucleic acid encoding a test biomolecule in the cell.

Another aspect of the invention provides a method of identifying a protein, protein region or protein: protein interaction (PPI) site which may be a useful target for the therapeutic modulation of a cell signalling pathway, the method comprising:
screening for a compound which inhibits a cell signalling pathway in accordance with the invention,
providing a test compound which causes a mammalian cell to express the first detectable reporter but not the second detectable reporter in the method in accordance with the invention,
identifying an intracellular binding partner which binds the test compound which causes a mammalian cell to express the first detectable reporter but not the second detectable reporter in the method in accordance with the invention, said binding partner being a candidate target protein for modulation of a cell signalling pathway,
preferably wherein the test compound which causes a mammalian cell to express the first detectable reporter but not the second detectable reporter in the method in accordance with the invention is a biomolecule which is encoded by a nucleic acid from a mammalian cell.

One or more transfected cells which express the first detectable reporter but not the second detectable reporter may be isolated.

The nucleic acid encoding the test biomolecule from said one or more transfected cells may be amplified, cloned and/or sequenced.

The nucleic acid encoding the test biomolecule may be expressed to produce the test biomolecule.

An intracellular binding partner which binds the test biomolecule may be identified, said binding partner being a candidate target protein for modulation of a cell signalling pathway.

The region of the intracellular binding partner which binds to the test biomolecule may be identified, said region being a candidate target region or site for modulation of a cell signalling pathway.

The target protein, protein region or protein: protein interaction (PPI) site may modulate a phenotypic response in a mammalian cell.

Another aspect of the invention provides a cultured mammalian cell or a population of mammalian cells, each cell comprising:
(a) a first heterologous nucleic acid comprising;
   (i) a nucleotide sequence encoding a first detectable reporter and,
   (ii) a constitutive regulatory element which is operably linked to the nucleotide sequence; and,
(b) a second heterologous nucleic acid comprising:
   (i) a nucleotide sequence encoding a repressor molecule which inhibits, inactivates or suppresses expression of the first detectable reporter,
   (ii) a nucleotide sequence encoding a second detectable reporter; and,
   (iii) a signal-activated regulatory element which is activated by said cell signalling pathway,
said signal-activated regulatory element being operably linked to the nucleotide sequence which encodes the repressor molecule and the nucleotide sequence which encodes the second detectable reporter,
wherein the expression of the first detectable reporter is fine-tuned by:
(a) regulating the expression of the first detectable reporter using promoters of differing strengths; and/or
(b) including an in-frame destabilisation nucleotide sequence within the nucleotide sequence of the first detectable reporter; and/or
(c) including a nucleotide sequence which targets proteins for proteosomal degradation in the nucleotide sequence encoding a first detectable reporter; and/or
(d) altering the stability of the first detectable reporter by incorporating adenylate-uridylate-rich elements (ARE's) into the 3' untranslated region (UTR) of its messenger RNA,
preferably which is transfected with a library of nucleic acids encoding a diverse population of test biomolecules,
preferably wherein the repressor molecule is miRNA.

In some embodiments, the cell or population may be transfected with a nucleic acid encoding a test biomolecule or a library of nucleic acids encoding a diverse population of test biomolecules, respectively.

The advantage provided by the invention described herein is that it allows the full complexity of the proteome to be screened for its function in a live cell 'phenotypic' assay format with peptide libraries (or RNAi, or genome editing libraries) comprising 10⁹ sequence diversity. This method permits strong positive selection and the isolation of a small number of true hit peptides (or nucleic acids) from a very large number of non-hit peptide (or nucleic acid) sequences enabling a clear linkage with disease to be established. The advantage of screening peptide libraries is that they, like small molecule drugs, typically act by directly and acutely inhibiting target function (rather than eliminating the target's long-term expression), the key to this method is turning a 'negative' cell phenotype signal (e.g., shut down of a signalling pathway or disruption of a protein/protein interaction) into a positive signal that can be selected for, or isolated from a large pool of cells harbouring inactive peptide sequences.

The identification of targets and their druggable sites that can be directly linked to disease, in a precise, efficient and reliable manner offers clear advantages in the development of suitable drug candidates for the treatment of disease. In particular, the method of the invention provides for the identification of intracellular targets and key druggable sites that play a role in disease progression, that may not otherwise be identifiable in other assay formats such as gene knock-out and RNA knock-down studies.

Another aspect of the invention provides a vector or combination of vectors which comprises:
(a) a first heterologous nucleic acid comprising;
   (i) a nucleotide sequence encoding a first detectable reporter and,
   (ii) a constitutive regulatory element which is operably linked to the nucleotide sequence; and,
(b) a second heterologous nucleic acid comprising:
   (i) a nucleotide sequence encoding an repressor molecule which inhibits, inactivates or suppresses expression of the first detectable reporter,
   (ii) a nucleotide sequence encoding a second detectable reporter; and,
   (iii) a signal-activated regulatory element which is activated by said cell signalling pathway,
said signal-activated regulatory element being operably linked to the nucleotide sequence which encodes the repressor molecule and the nucleotide sequence which encodes the second detectable reporter,
wherein the expression of the first detectable reporter is fine-tuned by:
(a) regulating the expression of the first detectable reporter using promoters of differing strengths; and/or
(b) including an in-frame destabilisation nucleotide sequence within the nucleotide sequence of the first detectable reporter; and/or
(c) including a nucleotide sequence which targets proteins for proteosomal degradation in the nucleotide sequence encoding a first detectable reporter; and/or
(d) altering the stability of the first detectable reporter by incorporating adenylate-uridylate-rich elements (ARE's) into the 3' untranslated region (UTR) of its messenger RNA,
preferably wherein the repressor molecule is miRNA.

### Brief Description of the Drawings

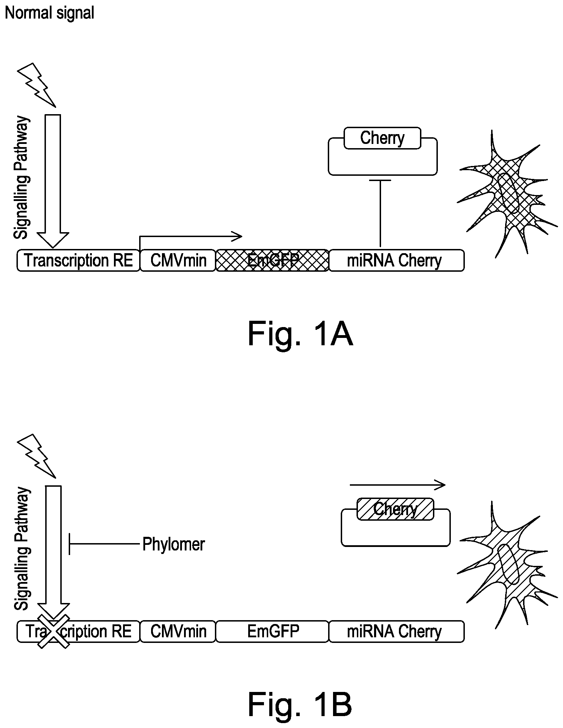

Figure 1 shows a schematic representation of a screening method according to an embodiment of the invention. Figure 1A shows that activation of the signalling pathway leads to activation of the transcriptional response element, which drives expression of EmGFP and the miRNA which then blocks the expression of the Cherry reporter. In this scenario, a cell would fluoresce green. Figure 1B shows that, when the signalling pathway is activated, but is blocked by an inhibitor, the transcriptional response element is not activated. In this case, neither emGFP nor the miRNA which blocks Cherry are activated. This allows the expression of Cherry and the cell fluoresces red.

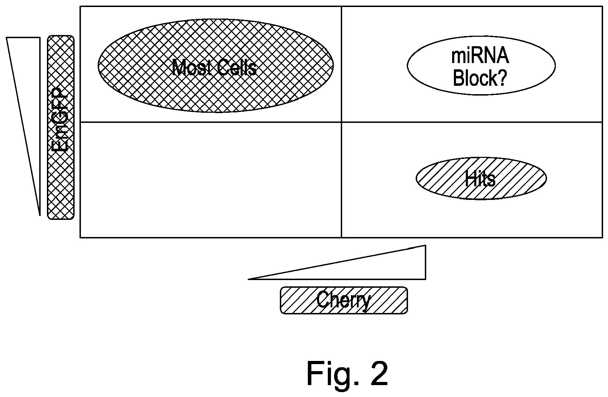

Figure 2 shows a summary of the FACS phenotypes generated by screening methods according to some embodiments of the invention.
Figure 3 shows different vectors tested in HeLa cells. Cherry constructs transiently transfected in HeLa cells, scanned live on the Arrayscan 01Sep11. Intensities can be directly compared.
Figure 4 shows different vectors tested in C3H10T1/2 cells.
Figure 5 shows the effect of ARE on mCherry plasmid stability in transiently transfected U2OS cells.

### Detailed Description of Invention

This invention relates to cell-based screening methods for the identification of biomolecules which inhibit cell-signalling pathways and the identification of proteins and surface sites of PPIs on proteins which participate in signal transduction and may be useful as drug targets to modulate cell-signalling pathways, in particular pathways which are active in cancer cells.

A cell signalling pathway is a series of interacting factors in a cell which transmits an intracellular signal within the cell in response to an extracellular stimulus at the cell surface and leading to changes in cell phenotype. Transmission of signals along a cell signalling pathway results in the activation of one or more transcription factors which alter gene expression. Preferred cell signalling pathways display aberrant activity, for example activation, up-regulation or mis-regulation in diseased cells, such a cancer cells. For example a pathway may be constitutively activated (i.e. permanently switched on) in a cancer cell, or inappropriately activated by an extracellular ligand, for example in an inflammatory cell in rheumatoid arthritis.

A functional cell signalling pathway is a pathway which is intact and capable of transmitting signals, if the pathway is switched on or activated, for example by an appropriate extracellular stimulus. An active cell signalling pathway is a pathway which has been switched on, for example by an appropriate extracellular stimulus and is actively transmitting signals.

Suitable cell signalling pathways include any signalling pathway which results in a transcriptional event in response to a signal received by a cell which results in a transcriptional event.

Cell signalling pathways for investigation as described herein may include cell signalling pathways which may be activated in cancer cells, such as Ras/Raf, Hedgehog, Fas, Wnt, Akt, ERK, TGFβ, and Notch signalling pathways.

Mammalian cells for use in the methods described herein may be any cultured mammalian cell, for example a human or non-human cell, in which the cell signalling pathway is functional.

During the screening methods described herein, the cell signalling pathway of interest is active in the mammalian cell or mammalian cell population. In the cell or cells, the active pathway activates the signal-activated regulatory element which causes expression of the second detectable reporter and the repressor molecule. The repressor molecule then suppresses expression of the first detectable reporter, such that the cells express the second detectable reporter, but not the first.

The cell signalling pathway of interest may be activated by any suitable technique.

In some embodiments, the cell signalling pathway of interest may be constitutively activated in the mammalian cell i.e. the signalling pathway is permanently switched on and active in the cell. For example, a mammalian cell may have a mutation, preferably in an upstream pathway component of the pathway, such as a cell surface receptor, which causes constitutive activation of the pathway. For example, Hedgehog signalling is constitutively active in osteosarcoma cell lines (Hirotsu et al. Molecular Cancer 2010, 9:5 http://www.molecular-cancer.com/content/9/1/5).

In other embodiments, the cell signalling pathway of interest may be activated in the mammalian cell by transfection of a mutant protein which causes constitutive activation. The activation of cell signalling pathways in cell lines through mutations is well known in the art. For example, Smoothened M2 mutants (SmoM2) are known to cause activation of signalling pathways in human cancer cells.

In other embodiments, the cell signalling pathway may be activated in the mammalian cell by an appropriate extracellular stimulus. For example, the cell may be treated with a ligand which activates the pathway by binding to a cell surface receptor. The ligand may be a drug or a natural ligand. For example, a cell may be treated with recombinant sonic hedgehog protein to activate hedgehog signalling.

Mammalian cells for use in the methods described herein comprise at least two heterologous nucleic acids (i.e. first and second heterologous nucleic acids).

A heterologous nucleic acid is a nucleic acid molecule which does not exist naturally in the mammalian cell. Heterologous nucleic acids may be recombinant or synthetic and may be introduced into a mammalian cell by any suitable molecular biology technique, such as transformation or transfection. A heterologous nucleic acid may be extra-chromosomal or may be incorporated into the genome of a mammalian cell.

A mammalian cell may comprise a first heterologous nucleic acid which comprises a first reporter coding sequence encoding a first detectable reporter.

A detectable reporter is a polypeptide which can be detected when it is expressed in the cell may be detected. For example, expression of the detectable reporter may lead to the production of a signal, for example a fluorescent, bioluminescent or colorimetric signal, which can be detected using routine techniques. The signal may be produced directly from the reporter, after expression, or indirectly through a secondary molecule, such as a labelled antibody.

Suitable detectable reporters include fluorescent proteins which produce a detectable fluorescent signal. Suitable fluorescent reporters are well known in the art and include Y66H, Y66F, EBFP, EBFP2, Azurite, GFPuv, T-Sapphire, TagBFP, Cerulean, mCFP, ECFP, CyPet, Y66W, dKeima-Red, mKeima-Red, TagCFP, AmCyan1, mTFP1 (Teal), S65A, Midoriishi-Cyan, Wild Type GFP, S65C, TurboGFP, TagGFP, TagGFP2, AcGFP1, S65L, Emerald, S65T, EGFP, Azami-Green, ZsGreen1, Dronpa-Green, TagYFP, EYFP, Topaz, Venus, mCitrine, YPet, TurboYFP, PhiYFP, PhiYFP-m, ZsYellow1, mBanana, Kusabira-Orange, mOrange, mOrange2, mKO, TurboRFP, tdTomato, DsRed-Express2, TagRFP, DsRed monomer, DsRed2 ("RFP"), mStrawberry, TurboFP602, AsRed2, mRFP1, J-Red, mCherry, HcRed1, mKate2, Katushka (TurboFP635), mKate (TagFP635), TurboFP635, mPlum, mRaspberry, mNeptune and E2-Crimson.

Suitable fluorescent reporters are available commercially (Clontech Labs Inc USA, Evrogen Moscow, RU; MBL Int MA USA; Addgene Inc MA USA).

Suitable detectable reporters also include cell surface markers which contain epitopes not otherwise present on the cell surface (i.e. unique epitopes). Expression of a cell surface marker may be detected using a labelled antibody which binds to the marker and produces a detectable signal.

Any cell surface marker which is not normally expressed on the mammalian cell may be used. For example, immune cell markers such as CD8 and CD19 may be employed, when the mammalian cells are not non-immune cells which do not express CD8 and CD19.

Other reporters include gene products (such as enzymes) whose expression in a mammalian cell can be detected with a live cell assay (i.e. an assay which does not require cell fixation or lysis). For example, a suitable reporter might include β-galactosidase, which can be detected using substrates such as CMFDG which fluoresces upon β-galactosidase-dependent catalysis.

The detectable reporter may comprise an in-frame destabilization sequence, for example a PEST sequence, within the coding sequence of the reporter. A PEST sequence is a peptide sequence that is rich in proline (P), glutamic acid (E), serine (S), and threonine (T).

The detectable reporter may further comprise a CL1 degron sequence at the C-terminal. CL1 degron sequence down regulates expression by specifically targets proteins for proteosomal degradation.

The detectable reporter may comprise an in-frame destabilization sequence, for example a PEST sequence and /or a CL1 degron sequence at the C-terminal.

The nucleotide sequence which encodes the first detectable reporter is operably linked to a constitutive regulatory element.

A regulatory element is a sequence of nucleotides from which transcription may be initiated of a nucleotide sequence operably linked downstream (i.e. in the 3' direction on the sense strand of double-stranded DNA).

A nucleotide sequence which is operably linked to a regulatory element is joined as part of the same nucleic acid molecule, suitably positioned and oriented for transcription to be initiated from the regulatory element.

A constitutive regulatory element causes the nucleotide sequence to be transcribed at a constant rate in the mammalian cell independently of the presence or absence of extracellular stimuli. Suitable constitutive regulatory elements for expression in mammalian cells are well-known in the art and include viral promoters, such as CMV, SV40, HSV-TK, UBC and EF-1α.

In particular embodiments of the invention, the constitutive regulatory element is selected from the CMV promoter and HSV-TK.

The nucleotide sequence encoding the first detectable reporter is transcribed at a constant rate in the mammalian cells. The first detectable reporter is therefore expressed in the mammalian cell at a constant level or substantially constant level in the absence of repressor molecule. Expression of the repressor molecule abolishes expression of the first detectable reporter, despite a constant rate of transcription from the coding sequence.

A mammalian cell may comprise a second heterologous nucleic acid which comprises a first nucleotide sequence encoding a second detectable reporter.

Suitable detectable reporters are described above.

The second detectable reporter is different from the first detectable reporter i.e. the first and second detectable reporters directly or indirectly produce signals which can be distinguished from each other. For example, the first and the second detectable reporters may be first and second fluorescent proteins which fluoresce at different wavelengths; first and second cell surface markers which bind to different antibodies or the first detectable reporter may be one of a fluorescent protein and a cell surface marker and the second detectable reporter may encode the other of a fluorescent protein and a cell surface marker.

Suitable reporter sequences and combinations of reporter sequences are well known in the art.

In some preferred embodiments, the first and second detectable reporters are fluorescent proteins which fluoresce at different wavelengths. Suitable fluorescent proteins are described above. The first and second detectable reporters may be any pair of fluorescent proteins whose emission wavelengths are sufficiently different to allow resolution, for example by flow cytometry. Suitable pairs of fluorescent proteins include Cherry and emGFP.

In other embodiments, the first and second detectable reporters are cell surface markers which bind to first and second labelled antibodies, for example, fluorescently-labelled antibodies. The first and second antibodies may have different labels to allow the expression of the first and second detectable reporters to be distinguished.

The second heterologous nucleic acid may comprise a second nucleotide sequence which encodes a repressor which inactivates, inhibits or suppresses expression of the first detectable reporter.

Suitable repressor molecules include peptides and polypeptides such as protein aptamers, such as phylomers and antibody molecules, such as domain antibodies, nanobodies or scFv, which inhibit or inactivate the first detectable reporter.

Suitable repressor molecules include RNA molecules, such as miRNA, RNAi, siRNA, shRNA, ribozyme or antisense RNA, which suppress the expression of the first detectable reporter.

For example, expression of the first detectable reporter may be inhibited using anti-sense or sense technology. The use of these approaches to down-regulate gene expression is now well-established in the art.

In one example the repressor molecule is miRNA e.g. miRNA Cherry.

Anti-sense oligonucleotides may be designed to hybridise to the complementary sequence of nucleic acid, pre-mRNA or mature mRNA, interfering with the production of the first detectable reporter so that its expression is completely or substantially completely prevented. In addition to targeting coding sequence of the first detectable reporter, anti-sense techniques may be used to target control sequences of the first detectable reporter, e.g. in the 5' flanking sequence, whereby the anti-sense oligonucleotides can interfere with expression control sequences. The construction of anti-sense sequences and their use is described for example in Peyman and Ulman, Chemical Reviews, 90:543-584, (1990) and Crooke, Ann. Rev. Pharmacol. Toxicol. 32:329-376, (1992).

Transcription of the anti-sense strand of the second nucleotide sequence yields RNA which is complementary to normal mRNA transcribed from the sense strand of the first detectable reporter. The complementary anti-sense RNA sequence binds with first detectable reporter mRNA to form a duplex, inhibiting translation of the first detectable reporter mRNA from the first detectable reporter into protein.

The complete sequence corresponding to the coding sequence in reverse orientation need not be used. For example fragments of sufficient length may be used. It is a routine matter for the person skilled in the art to screen fragments of various sizes and from various parts of the coding or flanking sequences of a gene to optimise the level of anti-sense inhibition. It may be advantageous to include the initiating methionine ATG codon, and perhaps one or more nucleotides upstream of the initiating codon. A suitable fragment may have about 14-23 nucleotides, e.g. about 15, 16 or 17.

An alternative to anti-sense is to use a copy of all or part of the first detectable reporter sequence inserted in sense, that is the same, orientation as the target gene, to achieve reduction in expression of the first detectable reporter by co-suppression; Angell & Baulcombe (1997) The EMBO Journal 16,12:3675-3684; and Voinnet & Baulcombe (1997) Nature 389: pg 553). Double stranded RNA (dsRNA) has been found to be even more effective in gene silencing than both sense and antisense strands alone (Fire A. et al Nature 391, (1998)). dsRNA mediated silencing is gene specific and is often termed RNA interference (RNAi).

RNA interference is a two-step process. First, dsRNA is cleaved within the cell to yield short interfering RNAs (siRNAs) of about 21-23nt length with 5' terminal phosphate and 3' short overhangs (∼2nt). The siRNAs target the corresponding mRNA sequence specifically for destruction (Zamore P.D. Nature Structural Biology, 8, 9, 746-750, (2001)).

The use of miRNA, RNAi, siRNA and shRNA molecules to suppress gene expression is well known in the art (Fire A, et al., 1998 Nature 391:806-811; Fire, A. Trends Genet. 15, 358-363 (1999); Sharp, P. A. RNA interference 2001. Genes Dev. 15, 485-490 (2001); Hammond, S. M., et al., Nature Rev. Genet. 2, 110-1119 (2001); Tuschl, T. Chem. Biochem. 2, 239-245 (2001); Hamilton, A. et al., Science 286, 950-952 (1999); Hammond, S. M., et al., Nature 404, 293-296 (2000); Zamore, P. D., et al., Cell 101, 25-33 (2000); Bernstein, E., et al., Nature 409, 363-366 (2001); Elbashir, S. M., et al., Genes Dev. 15, 188-200 (2001); WO0129058; WO9932619, and Elbashir S M, et al., 2001 Nature 411:494-498).

Another possibility is that transcription of the second nucleotide sequence on produces a ribozyme, able to cut nucleic acid at a specific site - thus also useful in influencing expression of the first detectable reporter. Background references for ribozymes include Kashani-Sabet and Scanlon, 1995, Cancer Gene Therapy, 2(3): 213-223, and Mercola and Cohen, 1995, Cancer Gene Therapy, 2(1), 47-59.

An RNA molecule such as a siRNA, dsRNA or miRNA may comprise a partial sequence of the first detectable reporter mRNA, for example at least 10 nucleotides, at least 15 or at least 20 nucleotides of the first detectable reporter sequence.

Suitable RNA molecules for down-regulation of the first detectable reporter may possible 85% or more, 90% or more, 95% or more or 100% sequence identity with a contiguous sequence of 10 to 40 nucleotides from the first detectable reporter mRNA sequence.

In some preferred embodiments, the second nucleotide sequence encodes a miRNA which suppresses expression of the first detectable reporter.

A miRNA is a short RNA molecule of typically 21-25 nucleotides which specifically hybridises to the first detectable reporter mRNA in a mammalian cell and inhibits the translation or degradation of the mRNA and thus the expression of the encoded protein (Brown, BD and Naldini, L. Nat. Rev. Genetics; 2009; 10; 578-585). miRNA may therefore be used to specifically suppress the expression of the first detectable reporter. Suitable miRNA molecules for the suppression of any specific detectable reporter may be designed and produced using techniques which are well-known in the art (e.g. S. Ossowski, R et al Plant J. 53 (2008) 674-690; John et al, PLoS Biology, 11(2), 1862-1879, 2004). Various web-based tools are available to design primers to any specific target gene, including detectable reporter genes (e.g. WMD3 Web MicroRNA Designer Ossowski et al Max Planck Institute for Developmental Biology, Tübingen http://wmd3.weigelworld.org/cgi-bin/webapp.cgi?page=Help).

For example, a miRNA for the suppression of Cherry expression may have the nucleotide sequence according to SEQ ID No: 1

The mRNA of the reporter protein may comprise AU-Rich Elements (Adenylate-uridylate-rich elements or AREs) into the 3' UTR (3' untranslated region) of its mRNA. AREs are regions that contain repeated adenine and uridine bases and are a key factor in determining the stability of mRNA in mammalian cells (Chen, Chyi-Ying A et al, 1995, Trends in Biochemical Science, 20 (11): 465-470).

The sequences encoding the second detectable reporter and the miRNA are operably linked to a signal-activated regulatory element. In other words, the same signal-activated regulatory element initiates transcription of both the coding sequences (i.e. the sequences are co-cistronic).

A signal-activated regulatory element is a regulatory element which initiates transcription of operably linked coding sequences when a cell signalling pathway of interest is actively signalling (i.e. the pathway is active) and does not initiate transcription of operably linked coding sequences when the cell signalling pathway is not actively signalling (i.e. the pathway is inactive). A pathway may be inactive if it is not switched on, for example due to the absence of an appropriate extracellular stimulus, or if one or more steps or components of the pathway are blocked or inhibited.

For example, active signalling through a cell signalling pathway may lead to the activation of a transcription factor in the mammalian cell. Once activated, the transcription factor may then bind to one or more specific binding sites in the signal-activated regulatory element. This binding activates the regulatory element and switches on the transcription of the operably linked coding sequences.

The choice of signal-activated regulatory element will depend on the cell signalling pathway and the transcription factors which is activated by the pathway. For example, the Notch signalling pathway activates the transcription factor RBP-Jkappa (aka CBF-1) which binds to the nucleotide sequence GTGGGAA. A Notch signal-activated regulatory element may therefore comprise one or more copies of this sequence. The Wnt signalling pathway activates the transcription factor LEF1 which binds to the nucleotide sequence AGATCAAAGG. A Wnt signal-activated regulatory element may therefore comprise one or more copies of this sequence.

A signal-activated regulatory element may comprise one or more transcription factor binding sites linked to a minimal promoter sequence, such as a CMV minimal promoter.

Suitable signal-activated regulatory elements may be selected from the CMV promoter and the HSV-TK promoter, for example, to drive expression.

Nucleic acid as described herein may be readily prepared by the skilled person using standard techniques (for example, see Molecular Cloning: a Laboratory Manual: 3rd edition, Sambrook and Russell (2001) Cold Spring Harbor Laboratory Press; Molecular Biology, Second Edition, Ausubel et al. eds. John Wiley & Sons, 1992; Recombinant Gene Expression Protocols Ed RS Tuan (Mar 1997) Humana Press Inc). For example, first and second heterologous nucleic acids may be prepared by conventional solid phase synthesis techniques or may be produced by recombinant means.

The first and second heterologous nucleic acids may be comprised within the same or separate vectors. Separate vectors may be the same or different. Suitable vectors can be chosen or constructed, containing appropriate regulatory sequences, including promoter sequences for driving transcription of the coding nucleotide sequence, terminator fragments, polyadenylation sequences, enhancer sequences, marker genes and other sequences as appropriate, for expression in mammalian cells as described herein. Suitable vectors for expressing nucleic acid in mammalian cells are well known. For example, vectors may be plasmids, or viral vectors, such as adenovirus, adeno-associated virus, retrovirus (such as HIV, MLV and pMX-based vectors), lentivirus or alpha-virus vectors.

When the cell signalling pathway is active, the signal-activated regulatory element in the second heterologous nucleic acid in the mammalian cell is switched on and initiates transcription of the nucleotide sequences encoding the second detectable reporter and the repressor molecule. The mammalian cell therefore expresses the second detectable reporter and the repressor molecule, when the cell signalling pathway is active. Expression of the repressor molecule prevents expression of the first detectable reporter in the cell. The mammalian cell does not therefore express the first detectable reporter when the cell signalling pathway is active. In other words, when the cell signalling pathway is active, the mammalian cells express the second but not the first detectable reporter.

When the cell signalling pathway is inactive, for example when one or more steps or components are blocked or inhibited, the signal-activated regulatory element in the second heterologous nucleic acid in the mammalian cell is not switched on and there is no transcription of the nucleotide sequences encoding the second detectable reporter and the repressor molecule. The mammalian cell does not therefore express either the second detectable reporter or the repressor molecule, when the cell signalling pathway is inactive. The absence of repressor molecule expression means there is no suppression or inhibition of the first detectable reporter in the cell. The mammalian cell therefore expresses the first detectable reporter, when the cell signalling pathway is inactive. In other words, when the cell signalling pathway is inactive, the mammalian cells express the first but not the second detectable reporter.

When appropriately stimulated or constitutively activated, the cell signalling pathway may be inactive in the presence of a compound, such as a biomolecule, which inhibits a component of the pathway or blocks or inhibits a step in the pathway, for example by inhibiting a protein:protein interaction.

Detection of the expression of the first and second detectable reporters in the mammalian cell (i.e. the reporter phenotype) is therefore indicative of the inhibition of the cell signalling pathway. A mammalian cell which is negative for the first detectable reporter and positive for the second detectable reporter has an active signalling pathway and a cell which is positive for the first detectable reporter and negative for the second detectable reporter has an inactive (e.g. a blocked or inhibited) signalling pathway.

When a test compound, such as a biomolecule, is introduced to the mammalian cell under conditions in which the cell signalling pathway is normally active, the expression of the first detectable reporter without expression of the second detectable reporter is indicative that the compound is an inhibitor of the cell signalling pathway.

Suitable test molecules include small organic molecules (i.e. non-polymeric organic molecules with a molecular weight of less than 800 Da) or biomolecules, such as peptide and nucleic acid aptamers, antibody molecules, such as domain antibodies, nanobodies or scFv, and suppression RNA, such as RNAi, siRNA, shRNA, ribozyme or antisense RNA.

Test biomolecules encoded by a heterologous nucleotide sequence may be used in the methods described herein.

Preferably, the test biomolecule is a peptide aptamer.

A peptide aptamer is a short peptide sequence, for example 15 to 80 amino acids which binds specifically to a site on a target protein. In some embodiments, a peptide aptamer is contained within a stable scaffold protein, for example, a non-immunoglobulin scaffold such as fibronectin (adnectin™), ankyrin (DARPin™, lipocalin (anticalin™), trinectin, a kunitz domain, transferrin, nurse shark antigen receptor or sea lamprey leucine-rich repeat protein (Binz et al Nat Biotech 23 1257-1268 (2005)).

The sequence of a peptide aptamer may be fully or partially random or may be non-random.

In preferred embodiments, the peptide aptamer is a phylomer. A phylomer is a peptide of 15 to 80 amino acids, preferably 15 to 50 amino acids, encoded by a short fragment of nucleotide sequence, for example 45 to 240 nucleotides, from a microbial nucleic acid.

Microbial nucleic acid used to generate phylomers may include genomic DNA, RNA or cDNA obtained from one or more different micro-organisms, such as bacteria, Archaea or lower eukaryotes. Phylomers may be encoded by any reading frame of a fragment of nucleotide sequence. Preferably, the phylomer is encoded by a natural open reading frame (ORF) of the nucleotide sequence.

Phylomers and phylomer libraries are known in the art (Watt et al (2006) Nat Biotech 24 17-183; Watt et al (2006) Expert Opin Drug Disc 1 491-502, Watt et al (2009) Future Med Chem 1 (2) 257-265, WO2005/119244; WO/2004/074479, and, WO/2006/017913).

A phylomer library is a population of phylomers having diverse sequences. For example, a phylomer library may comprise 3 x 10⁴ or more, 1 x 10⁵ or more, 1 x 10⁶ or more, 1 x 10⁷ or more, 1 x 10⁸ or more different phylomer sequences, preferably 1 x 10⁸ to 1 x 10⁹.

Phylomer libraries may be constructed using any convenient technique. For example, a phylomer library may be constructed by randomly cloning short fragments of nucleotide sequence from one or more microbial nucleic acids into expression vectors. A phylomer library may be produced by a method comprising;
(i) producing fragments from nucleic acids from two or more microorganisms;
(ii) inserting the nucleic acid fragments into an expression vector adapted to express the fragment; and
(iii) expressing the peptide encoded by the nucleic acid fragment.

The nucleic acid fragments may be produced from genomic DNA, cDNA, or amplified nucleic acid from one or more microbial genomes or transcriptomes, preferably genomes.

The nucleic acid fragments may be produced from a mixture of nucleic acids (i.e. genomes or transcriptomes) from different microorganisms. The nucleic acids may be present in the mixture in an amount that is proportional to the complexity and size of the genome (or transcriptome), for example, in comparison to the complexity and size of other genomes in the mixture. This results in approximately equal representation of the genome fragments.

Preferably, a library of phylomers is produced from a mixture of two or more phylogenetically diverse microbial genomes or transcriptomes, including for example, genomic DNA of cDNA from extremophiles, for example thermophilic Archea and bacteria, such as *Archaeoglobus fulgidus, Aquifex aeolicus, Aeropyrum pernix, Pyrococcus horikoshii, Synechocystis* PCC 6803, *Thermoplasma volcanium, Thermotoga maritima, Thermus thermophilus, Methanobacterium thermoautotrophicum, Methanococcus jannaschii* and *Deinococcus radiodurans.*

Examples of phylomer libraries are described in EP1696038.

Nucleic acid fragments may be generated from one, two or more microbial genomes or transcriptomes by one or more of a variety of methods known to those skilled in the art. Suitable methods include, for example, mechanical shearing (e.g. by sonication or passing the nucleic acid through a fine gauge needle), digestion with a nuclease (e.g. Dnase 1), digestion with one or more restriction enzymes, preferably frequent cutting enzymes that recognize 4-base restriction enzyme sites and treating the DNA samples with radiation (e.g. gamma radiation or ultra-violet radiation). In some embodiments, nucleic acid fragments may be generated from one, two or more microbial genomes or transcriptomes by polymerase chain reaction (PCR) using, for example, random or degenerate oligonucleotides. Random or degenerate oligonucleotides may include restriction enzyme recognition sequences to allow for cloning of the amplified nucleic acid into an appropriate nucleic acid vector.

Each fragment of microbial nucleic acid produced as described above encodes a phylomer. The fragments may be cloned into expression vectors for expression of the phylomer.

Nucleic acid encoding a test biomolecule may be flanked (for example 5' and 3' to the coding sequence) by specific sequence tags. Sequence tags comprise 10 to 50 nucleotides of known sequence which may be used as binding sites for oligonucleotide primers. Preferably, the sequence of the tag is not found in the mammalian genome. This allows the coding sequence of a test biomolecule to be conveniently amplified from the mammalian cell, for example by PCR, if required.

The nucleic acid encoding the test biomolecule may be operably linked to a regulatory element and comprised in a vector for expression in the mammalian cell. Suitable regulatory elements and vectors are well-known in the art and described elsewhere herein. Suitable techniques for producing and manipulating nucleic acid and expressing it in mammalian cells are well-known in the art.

A method described herein may comprise transfecting a mammalian cell comprising first and second heterologous nucleic acids as described above with a nucleic acid encoding a test biomolecule to produce a transfected cell comprising a nucleic acid which encodes the test biomolecule.

In preferred embodiments, a population of mammalian cells is transfected with a library of nucleic acids encoding a diverse population of test biomolecules. A method described herein may comprise transfecting a population of mammalian cells comprising first and second heterologous nucleic acids as described above with a library of nucleic acids encoding a diverse population of test biomolecules to produce a population of transfected cells, each cell containing a nucleic acid which encodes a test biomolecule.

The library may be pooled to allow simultaneous transfection and screening of all the members of the library.

A suitable library of nucleic acids may, for example, encode 3 x 10⁴ or more, 1 x 10⁵ or more, 1 x 10⁶ or more, 1 x 10⁷ or more, 1 x 10⁸ or more different test biomolecules.. A library may for example encode 10⁸ to 10⁹ different test biomolecules.

The nucleic acids in the library are preferably cloned into vectors for mammalian cell expression as described above.

After transfection of the mammalian cells and expression of the nucleic acids encoding the test biomolecules, the population of cells may be screened for expression of the first and second detectable reporters as described above to identify cells in which the test biomolecule has inhibited the cell signalling pathway.

As described above, expression of the first detectable reporter in a mammalian cell in the population the absence of expression of the second reporter is indicative that the test compound introduced to the mammalian cell, for example, a test biomolecule expressed by the cell, is an inhibitor of the cell signalling pathway.

Suitable techniques for determining reporter expression will depend on the reporters which are used. Typically, the detectable reporters will be fluorescent proteins or markers detectable with fluorescently labelled antibodies. The fluorescence phenotype of the cells in the population may be determined by any convenient fluorescent techniques.

If one or both of the detectable reporters are cell surface markers, expression may be determined by contacting the population of cells with antibodies which bind to the reporters. The antibodies may be labelled, for example with a fluorescent label. If both detectable reporters are cell surface markers, the antibodies which bind to each marker may be labelled with fluorescent labels which are distinguishable. After labelling, the amount of label attached to each cell may be determined, for example by measuring fluorescence, to determine the expression of the reporter.

Mammalian cells with the desired phenotype of reporter expression may be isolated. For example, cells which express the first detectable reporter but not the second detectable reporter may be isolated from other cells in the population.

In some preferred embodiments, fluorescence activated cell sorting (FACS) may be used to detect reporter expression and isolate cells with a defined fluorescence phenotype, for example cells which are positive for the first detectable reporter and negative for the second detectable reporter. Suitable FACS techniques are well known in the art. (see for example Ormerod, M.G. (1999) Flow Cytometry. 2nd edition. BIOS Scientific Publishers, Oxford. ISBN 185996107X).

Any suitable FACS apparatus may be employed (e.g. MoFlo Astrios Cell Sorter (Beckman Coulter Inc, CA USA).

As described above, the test compound introduced to mammalian cells which are found to be positive for the first detectable reporter and negative for the second detectable reporter is a putative inhibitor of the cell signalling pathway. For example, a test biomolecule which is expressed in mammalian cells which are positive for the first detectable reporter and negative for the second detectable reporter is a putative inhibitor of the cell signalling pathway.

Nucleic acids encoding test biomolecules may be isolated from mammalian cells which are positive for the first detectable reporter and negative for the second detectable reporter.

Techniques for the isolation of nucleic acid from a mammalian cell are well-known in the art. For example, total DNA may be isolated from the cells and the nucleic acid encoding the test biomolecule may then be amplified from the isolated total DNA. In some preferred embodiments, the nucleic acid may be amplified using primers which hybridise to the sequence specific tags flanking the test biomolecule coding sequence.

Nucleic acids encoding test biomolecules or amplification products thereof may be cloned into vectors and/or sequenced.

Sequencing may be useful, for example, in identifying and distinguishing individual test biomolecule coding sequences from the population of mammalian cells positive for the first detectable reporter and negative for the second detectable reporter.

The population of test nucleic acids isolated from mammalian cells positive for the first detectable reporter and negative for the second detectable reporter in a first screen, or amplification products thereof, may be used in one or more further rounds of screening as described above to generate a sub-population which is enriched for sequences encoding inhibitory biomolecules. For example, a method may comprise;
(5) providing a further population of mammalian cells comprising a first and a second heterologous nucleic acid, as described in step (1) of the first aspect of the invention;
(6) transfecting said further population of cells with said population of nucleic acids encoding test biomolecules isolated from the one or more mammalian cells positive for the first detectable reporter and negative for the second detectable reporter,
(7) expressing the population of nucleic acids in the further population of said mammalian cells, and
(8) determining the expression of the first and the second detectable reporters in the mammaliancells,
   wherein expression of the first detectable reporter but not the second detectable reporter is indicative that the test biomolecule expressed by a nucleic acid in a mammalian cell is an inhibitor of said cell signalling pathway,
(9) identifying one or more mammalian cells in the further population which express the first detectable reporter but not the second detectable reporter, and
(10) isolating the one or more mammalian cells which express the first detectable reporter but not the second detectable reporter,
optionally comprising isolating nucleic acids encoding test biomeolecules from the one or more mammalian cells isolated in step (10),
optionally wherein steps (5) to (10) are repeated one or more times.

One, two, three or more rounds of screening may be performed until one or more nucleic acids encoding inhibitory test biomolecules have been identified.

In some embodiments, the identified nucleic acids may be further manipulated, for example by re-cloning. In some embodiments, the nucleic acid may be cloned into an expression vector adjacent to nucleic acid encoding a heterologous peptide, such that the vector expresses a fusion protein comprising the biomolecule fused to the heterologous peptide. Suitable heterologous peptides include epitope tags, affinity tags and cell penetrating peptides (CPPs).

An epitope tag is a heterologous amino acid sequence which forms one member of a specific binding pair. Peptides containing an epitope tag may be isolated and/or detected through the binding of the other member of the specific binding pair to the epitope tag. For example, the epitope tag may be an epitope which is bound by an antibody molecule. Suitable epitope tags are well-known in the art including, for example, MRGS(H)₆, DYKDDDDK (FLAG™), T7-, S- (KETAAAKFERQHMDS), poly-Arg (R5-6), poly-His (H2-10), poly-Cys (C4) poly-Phe(Fl 1) poly-Asp(D5-16), Strept-tag II (WSHPQFEK), c-myc (EQKLISEEDL), Influenza-HA tag (Murray, P. J. et al (1995) Anal Biochem 229, 170-9), Glu-Glu-Phe tag (Stammers, D. K. et al (1991) FEBS Lett 283, 298-302), Tag.100 (Qiagen; 12 aa tag derived from mammalian MAP kinase 2), Cruz tag 09™ (MKAEFRRQESDR, Santa Cruz Biotechnology Inc.) and Cruz tag 22™ (MRDALDRLDRLA, Santa Cruz Biotechnology Inc.). Other suitable tags include GST (glutathione-S-transferase), MBP (maltose binding protein), GAL4, β-galactosidase, biotin and strepavidin. Known tag sequences are reviewed in Terpe (2003) Appl. Microbiol. Biotechnol. 60 523-533.

Epitope tags may be useful in purifying and/or isolating the phylomer, for example for the immunoprecipitation of test biomolecules bound to cellular binding partners.

A CPP is a heterologous amino acid sequence which facilitates transport of an attached moiety across a cell membrane. Suitable CPPs are well-known in the art including, basic peptides, such as Drosophila homeoprotein antennapedia transcription protein (AntHD), HSV structural protein VP22, HIV TAT protein, Kaposi FGF signal sequence (kFGF), protein transduction domain-4 (PTD4), Penetratin, M918, Transportan-10, PEP-I peptide, nuclear localization sequences, amphipathic peptides, and peptide sequences comprising 5 or more contiguous basis residues, such as arginines or lysines (e.g. (R)₉, (K)₉, (R)₁₁, or (K)₁₁). Other suitable CPPs are known in the art (see for example Inoue et al., 2006 Eur. Urol. 49, 161-168; Michiue et al., 2005 J. Biol. Chem. 280, 8285-8289; Wadia and Dowdy, 2002 Curr. Opin. Biotechnol. 13 52-56; Langel (2002) Cell Penetrating Peptides, CRC Press, Pharmacology and Toxicology Series; US6730293, WO05/084158 and WO07/123667)). Wadia & Dowdy Current Opin Biotechnology (2002) 13 52-56; Wagstaff & Jans Curr Medicinal Chemistry 13 1371-1387 (2006).

CPPs may be useful in transporting a test biomolecule into a cell, for example to screen directly for effects on cell phenotype.

A test biomolecule which alters cellular phenotype, optionally fused to an epitope tag and/or a CPP, may be produced or synthesised.

Various approaches for the production of biomolecules are available. Encoding nucleic acid maybe expressed to produce the test biomolecule (see for example, Recombinant Gene Expression Protocols Ed RS Tuan (Mar 1997) Humana Press Inc). Alternatively, test biomolecules may be generated wholly or partly by chemical synthesis. Test biomolecules may be synthesised using liquid or solid-phase synthesis methods; in solution; or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulfonic acid or a reactive derivative thereof. Chemical synthesis of peptides is well-known in the art (J.M. Stewart and J.D. Young, Solid Phase Peptide Synthesis, 2nd edition, Pierce Chemical Company, Rockford, Illinois (1984); M. Bodanzsky and A. Bodanzsky, The Practice of Peptide Synthesis, Springer Verlag, New York (1984); J. H. Jones, The Chemical Synthesis of Peptides. Oxford University Press, Oxford 1991; in Applied Biosystems 430A Users Manual, ABI Inc., Foster City, California; G. A. Grant, (Ed.) Synthetic Peptides, A User's Guide. W. H. Freeman & Co., New York 1992, E. Atherton and R.C. Sheppard, Solid Phase Peptide Synthesis, A Practical Approach. IRL Press 1989 and in G.B. Fields, (Ed.) Solid-Phase Peptide Synthesis (Methods in Enzymology Vol. 289). Academic Press, New York and London 1997).

Having identified a test biomolecule which alters cellular phenotype and produced the test biomolecule, optionally as a fusion protein, a method may further comprise confirming the effect of the test biomolecule on the phenotype of a mammalian cell. For example, test biomolecules which have been synthesised with a Cell-Penetrating Peptide (CPP) may be used directly on the cells in order to elicit a phenotypic deflection.

Inhibitory biomolecules expressed from test nucleic acids identified from a library as described above may be used to screen for intracellular binding partners, for example cellular proteins which bind to the biomolecule. For example, the expressed biomolecule may be used as a bait molecule to identify intracellular binding partners in a mammalian cell or cell extract. Cellular proteins which bind to the bait biomolecule may be isolated.

Suitable techniques for identifying intracellular binding partners are well known in the art. They include techniques such as radioimmunoassay, co-immunoprecipitation, scintillation proximity assay and ELISA methods. For example, the biomolecule may be over-expressed in mammalian cells, immunoprecipitated with antibodies binding to the epitope tag. Proteins bound to the biomolecule may be analysed, for example by MALDI-linked TOF mass spectrometry, and identified.

A method may comprise identifying a cellular binding partner of a test biomolecule identified in a screen described above. For example, the cellular binding partner may be a protein which specifically interacts with or binds to the test biomolecule.

Since the test biomolecule inhibits a cellular signalling pathway, the cellular binding partner may be identified as a putative component of the pathway. This may be useful as a target for the development of therapeutics which modulate the pathway.

Following identification of the cellular binding partner, the binding site, region or domain of the cellular binding partner which interacts with the test biomolecule may be identified. This site region or domain may also be useful as a target site for the development of therapeutics which modulate the pathway.

For example, X ray crystallography, NMR or standard biochemical techniques, such as immunoprecipitation, based on series of deletion constructs may be performed. For example, test biomolecules may be co-crystallised with the target protein and the structure solved.

Following identification of a target protein by a method described herein, the interaction site of the target protein may be investigated.

The interaction site is the site or region at which the bait biomolecule binds to block the activity of the target protein. Since binding at the interaction site blocks activity, the interaction site is the site or region of a target protein through which the target protein binds to a binding partner. For example, the interaction site may be the site of a protein: protein interface when the target protein is bound to its binding partner.

Blockade of the interaction site e.g. by a small organic molecule, antibody or other biomolecule which binds at the site may disrupt binding of the target protein to a binding partner. Binding at the interaction site may therefore modulate the activity of the target protein and alter one or more phenotypic traits or characteristics.

Methods of the invention may further comprise screening for test compounds, such as small organic molecule, antibodies, nucleic acids or peptides, which bind to the same interaction site on a target protein as a biomolecule identified as described above.

Conventional techniques, such as displacement assays may be employed, to screen for compounds which compete with the biomolecule for binding to the target protein. For example, a method may comprise contacting a complex comprising the target protein bound to the biomolecule with a test compound. Displacement of the biomolecule by the test compound is indicative that test compound binds to the target protein at the same site as the phylomer. Standard displacement assay platforms, such as Alpha-LISA™ or fluorescence polarisation, may be employed.

Further displacement assays may be performed using truncated versions of the biomolecule in order to determine the key binding determinants in the binding interface. High throughput small molecule displacement screens may then be performed using a fluorescently-labelled version of these 'minimised' biomolecules.

Small molecules which can displace the test compound from the target protein are predicted to also inhibit the activity of the target protein in a cell, and may be useful in the development of therapeutics.

Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above and tables described below.

### Examples

### Example 1

In order to tune the levels of miRNA and reporter proteins in the screening system to generate an appropriate signal window, a number of approaches were adopted. The presence of residual reporter protein in the screening system, which may have remained in the cells for some time after it has been translated, can mask the detection of mCherry reporter protein and so give sub-optimal results. The following studies were carried out to optimise the detection of the mRNA and reporter protein so that the read-out is enhanced and the method is able to detect signals to a significantly higher degree of accuracy.

A representative list of vectors we have used to address these issues is shown in Table 1 below.

**Table 1**

| **Vector number** | **Vector name** | **Promoter** | **Destabilisation** |
|---|---|---|---|
| 192 | pmC4.10CMV | CMV | No destabilisation |
| 193 | pmC4.11 CMV | CMV | PEST destabilisation sequence |
| 194 | pmC4.22CMV | CMV | CL1 and PEST destabilisation sequences |
| 195 | pmC4.11 HSV-TK | HSV-TK | PEST destabilisation sequence |
| 196 | pmC4.22HSV-TK | HSV-TK | CL1 and PEST destabilisation sequences |
| 197 | pmC4.10CMV-PURO | CMV | No destabilisation |
| 198 | pmC4.11CMV-PURO | CMV | PEST destabilisation sequence |
| 199 | pmC4.11CMV-TK-PURO | HSV-TK | PEST destabilisation sequence |

In this series of vectors, mCherry expression levels were regulated by using promoters of differing strengths. For example the human cytomegalovirus (CMV) promoter or the HSV-TK promoter. Further regulation of reporter protein expression has been achieved by including an in-frame destabilization sequence, for example a PEST sequence, within the coding sequence of the reporter.

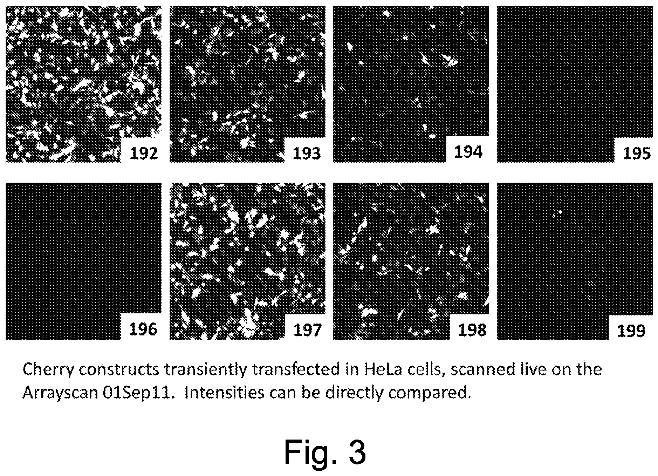

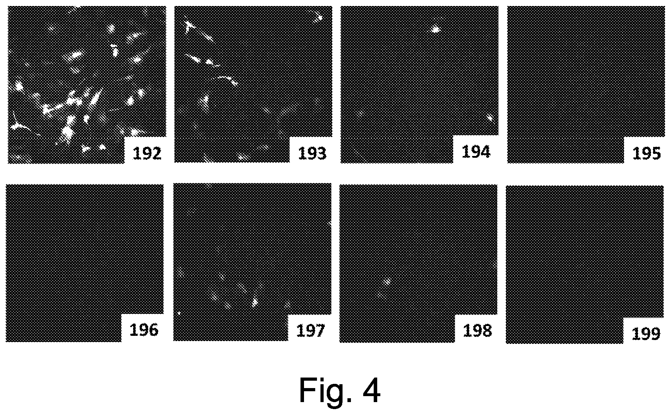

Reporters were further down-regulated by the inclusion of a CL1 degron sequence at the C-terminal, a sequence which specifically targets proteins for proteosomal degradation. As shown in Figures 3 and 4, by transfection of vectors into two different cell types, these different approaches lead to very different levels of reporter expression.

The results show that a first nucleic acid comprising a PEST sequence within the coding sequence of the reporter leads to significant decrease of expression.
A combination of PEST and CL1 degron at the C-terminus of the coding sequence provides a further reduction of expression, thereby improving the sensitivity and efficiency of the method described herein.

### Example 2

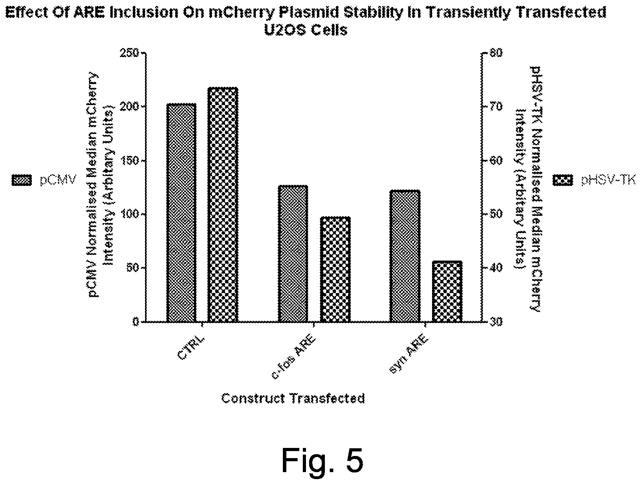

AU-Rich Elements (ARES) were incorporated into the 3' UTR of its mRNA. Figure 5 shows a comparison between mCherry reporters engineered to be unmodified (CTRL), to contain a naturally-occurring ARE (C-fos) or to contain a synthetic (Syn) ARE. The data shows the reduction in mCherry signal upon inclusion of an ARE using either the CMV or HSV-TK promoters to drive expression.

By using various combinations of promoters, destabilization sequences and mRNA degradation sequences, we can "fine-tune" the levels of miRNA and reporter protein so that they are appropriate for use in the screening system.

### Sequences

**SEQ ID No: 1**
5'TTGATGTTGACGTTGTAGGCGGTTTTGGCCACTGACTGACCGCCTACAGTCAACATCAA3'

## Claims

1. A method of screening for a compound which inhibits a cell signalling pathway comprising:
(1) providing a population of cultured mammalian cells having an active cell signalling pathway, each mammalian cell comprising:
(a) a first heterologous nucleic acid comprising;
(i) a nucleotide sequence encoding a first detectable reporter and,
(ii) a constitutive regulatory element which is operably linked to the nucleotide sequence; and,
(b) a second heterologous nucleic acid comprising:
(i) a nucleotide sequence encoding a repressor molecule which inhibits, inactivates or suppresses expression of the first detectable reporter,
(ii) a nucleotide sequence encoding a second detectable reporter; and,
(iii) a signal-activated regulatory element which is activated by said cell signalling pathway,
said signal-activated regulatory element being operably linked to the repressor nucleotide sequence and the nucleotide sequence which encodes the second detectable reporter,
(2) introducing a library of test compounds into said population of mammalian cells, and;
(3) determining the expression of the first and the second detectable reporters in one or more of the population of transfected cells,
wherein expression of the first detectable reporter but not the second detectable reporter in a transfected cell is indicative that the test compound inhibits said cell signalling pathway,
wherein the expression of the first detectable reporter is fine-tuned by:
(a) regulating the expression of the first detectable reporter using promoters of differing strengths; and/or
(b) including an in-frame destabilisation nucleotide sequence within the nucleotide sequence of the first detectable reporter; and/or
(c) including a nucleotide sequence which targets proteins for proteosomal degradation in the nucleotide sequence encoding a first detectable reporter; and/or
(d) altering the stability of the first detectable reporter by incorporating adenylate-uridylate-rich elements (ARE's) into the 3' untranslated region (UTR) of its messenger RNA,
optionally,
(4) identifying one or more cells in the population which express the first detectable reporter but not the second detectable reporter, wherein the test compound introduced into the one or more cells is a putative inhibitor of the cell signalling pathway,
preferably wherein the cell signalling pathway is constitutively activated in the mammalian cell,
preferably wherein the population of mammalian cells is provided by transfecting a population of mammalian cells with the first and second heterologous nucleic acids, preferably wherein the first and second heterologous nucleic acids are comprised within the same or separate expression vectors,
preferably wherein the repressor molecule is a miRNA.

2. A method according to claim 1 wherein the first and second detectable reporters are fluorescent proteins, preferably Cherry and emGFP respectively,
preferably wherein the expression of the first and second detectable reporters is determined by measuring the fluorescent emission of the reporters.

3. A method according to claim 1 or claim 2 wherein the test compounds are biomolecules, preferably peptides, more preferably phylomers.

4. A method according to claim 3 wherein the library of test compounds is introduced into the population of mammalian cells by expressing a library of nucleic acids encoding a diverse population of test biomolecules in said population,
preferably comprising the additional step before step (2) of transfecting the population of mammalian cells with the library of nucleic acids encoding the population of test biomolecules,
preferably wherein the library of nucleic acids encodes 1 x 10⁶ or more diverse test biomolecules,
preferably wherein the test biomolecules are peptides, more preferably phylomers.

5. A method according to claim 3 or claim 4 comprising isolating one or more mammalian cells which express the first detectable reporter but not the second detectable reporter, preferably wherein cells are isolated by fluorescence activated cell sorting.

6. A method according to claim 5 wherein the one or more isolated cells are cultured and/or expanded.

7. A method according to claim 5 or claim 6 comprising amplifying, cloning and/or sequencing the nucleic acid encoding the test biomolecule from said one or more mammalian cells.

8. A method according to any one of claims 5 to 7 comprising isolating nucleic acids encoding test biomolecules from the one or more mammalian cells which express the first detectable reporter but not the second detectable reporter.

9. A method according to claim 7 or claim 8 comprising
(5) providing a further population of mammalian cells comprising a first and a second heterologous nucleic acid according to step (1) of claim 1,
(6) transfecting said further population of cells with said population of nucleic acids encoding test biomolecules isolated from the one or more mammalian cells positive for the first detectable reporter and negative for the second detectable reporter,
(7) expressing the population of nucleic acids in the further population of said mammalian cells, and
(8) determining the expression of the first and the second detectable reporters in the mammalian cells,
wherein expression of the first detectable reporter but not the second detectable reporter is indicative that the test biomolecule expressed by a nucleic acid in a mammalian cell is an inhibitor of said cell signalling pathway,
(9) identifying one or more mammalian cells in the further population which express the first detectable reporter but not the second detectable reporter, and;
(10) isolating the one or more mammalian cells which express the first detectable reporter but not the second detectable reporter,
optionally comprising isolating nucleic acids encoding test biomolecules from the one or more mammalian cells isolated in step (10),
optionally wherein steps (5) to (10) are repeated one or more times.

10. A method according to claim 4 when the library of nucleic acids encodes 1 x 10⁶ or more diverse test biomolecules, and claims 5 to 9 comprising expressing a nucleic acid isolated from a mammalian cell which expresses the first detectable reporter but not the second detectable reporter to produce the test biomolecule.

11. A method of identifying a protein, protein region or protein: protein interaction (PPI) site which may be a useful target for the therapeutic modulation of a cell signalling pathway, the method comprising:
screening for a compound which inhibits a cell signalling pathway according to any one of the preceding claims,
providing a test compound which causes a mammalian cell to express the first detectable reporter but not the second detectable reporter in the method according to any one of the preceding claims,
identifying an intracellular binding partner which binds the test compound which causes a mammalian cell to express the first detectable reporter but not the second detectable reporter in the method according to any one of the preceding claims, said binding partner being a candidate target protein for modulation of a cell signalling pathway,
preferably wherein the test compound which causes a mammalian cell to express the first detectable reporter but not the second detectable reporter in the method according to any one of the preceding claims is a biomolecule which is encoded by a nucleic acid from a mammalian cell.

12. A method according to claim 11 when the test compound which causes a mammalian cell to express the first detectable reporter but not the second detectable reporter in the method according to any one of the preceding claims is a biomolecule which is encoded by a nucleic acid from a mammalian cell, the method comprising identifying a region of the intracellular binding partner which binds to the test biomolecule, said region being a candidate target region or site for modulation of a cell signalling pathway.

13. A method according to claim 11 or claim 12 wherein the target protein, protein region or protein: protein interaction (PPI) site modulates a phenotypic response in a mammalian cell.

14. A cultured mammalian cell or a population of mammalian cells, each cell comprising:
(a) a first heterologous nucleic acid comprising;
(i) a nucleotide sequence encoding a first detectable reporter and,
(ii) a constitutive regulatory element which is operably linked to the nucleotide sequence; and,
(b) a second heterologous nucleic acid comprising:
(i) a nucleotide sequence encoding a repressor molecule which inhibits, inactivates or suppresses expression of the first detectable reporter,
(ii) a nucleotide sequence encoding a second detectable reporter; and,
(iii) a signal-activated regulatory element which is activated by said cell signalling pathway,
said signal-activated regulatory element being operably linked to the nucleotide sequence which encodes the repressor molecule and the nucleotide sequence which encodes the second detectable reporter,
wherein the expression of the first detectable reporter is fine-tuned by:
(a) regulating the expression of the first detectable reporter using promoters of differing strengths; and/or
(b) including an in-frame destabilisation nucleotide sequence within the nucleotide sequence of the first detectable reporter; and/or
(c) including a nucleotide sequence which targets proteins for proteosomal degradation in the nucleotide sequence encoding a first detectable reporter; and/or
(d) altering the stability of the first detectable reporter by incorporating adenylate-uridylate-rich elements (ARE's) into the 3' untranslated region (UTR) of its messenger RNA,
preferably which is transfected with a library of nucleic acids encoding a diverse population of test biomolecules,
preferably wherein the repressor molecule is miRNA.

15. A vector or combination of vectors which comprises:
(a) a first heterologous nucleic acid comprising;
(i) a nucleotide sequence encoding a first detectable reporter and,
(ii) a constitutive regulatory element which is operably linked to the nucleotide sequence; and,
(b) a second heterologous nucleic acid comprising:
(i) a nucleotide sequence encoding an repressor molecule which inhibits, inactivates or suppresses expression of the first detectable reporter,
(ii) a nucleotide sequence encoding a second detectable reporter; and,
(iii) a signal-activated regulatory element which is activated by said cell signalling pathway,
said signal-activated regulatory element being operably linked to the nucleotide sequence which encodes the repressor molecule and the nucleotide sequence which encodes the second detectable reporter,
wherein the expression of the first detectable reporter is fine-tuned by:
(a) regulating the expression of the first detectable reporter using promoters of differing strengths; and/or
(b) including an in-frame destabilisation nucleotide sequence within the nucleotide sequence of the first detectable reporter; and/or
(c) including a nucleotide sequence which targets proteins for proteosomal degradation in the nucleotide sequence encoding a first detectable reporter; and/or
(d) altering the stability of the first detectable reporter by incorporating adenylate-uridylate-rich elements (ARE's) into the 3' untranslated region (UTR) of its messenger RNA,
preferably wherein the repressor molecule is miRNA.

16. A method as claimed in any of claims 1 to 13, a cell as claimed in claim 14 or a vector as claimed in claim 15 wherein the expression of the first detectable reporter is fine-tuned by (b), wherein the in-frame destabilisation sequence is a PEST sequence.

17. A method as claimed in any of claims 1 to 13 or 16, a cell as claimed in claim 14 or a vector as claimed in claim 15 wherein the expression of the first detectable reporter is fine-tuned by (c), wherein the nucleotide sequence which targets proteins for proteosomal degradation is a CL1 degron sequence.

## Patentansprüche

1. Verfahren zum Screening auf eine Verbindung, die einen Zellsignalisierungsweg hemmt, umfassend:
(1) Bereitstellen einer Population an kultivierten Säugetierzellen, die einen aktiven Zellsignalisierungsweg aufweisen, wobei jede Säugetierzelle Folgendes umfasst:
(a) eine erste heterologe Nukleinsäure, umfassend:
(i) eine Nukleotidsequenz, die einen ersten detektierbaren Reporter codiert, und
(ii) ein konstitutives regulatorisches Element, das mit der Nukleotidsequenz wirkverbunden ist; und
(b) eine zweite heterologe Nukleinsäure, umfassend:
(i) eine Nukleotidsequenz, die ein Repressormolekül codiert, das die Expression des ersten detektierbaren Reporters hemmt, deaktiviert oder unterdrückt,
(ii) eine Nukleotidsequenz, die einen zweiten detektierbaren Reporter codiert, und
(iii) ein signalaktiviertes regulatorisches Element, das durch den Zellsignalisierungsweg aktiviert wird,
wobei das signalaktivierte regulatorische Element mit der Repressornukleotidsequenz und der Nukleotidsequenz, die den zweiten detektierbaren Reporter codiert, wirkverbunden ist,
(2) Einführen einer Bibliothek von Testverbindungen in die Population an Säugetierzellen, und
(3) Bestimmen der Expression des ersten und des zweiten detektierbaren Reporters in einer oder mehreren aus der Population an transfizierten Zellen,
wobei Expression des ersten detektierbaren Reporters, aber nicht des zweiten detektierbaren Reporters in einer transfizierten Zelle darauf hinweist, dass die Testverbindung den Zellsignalisierungsweg hemmt,
wobei die Expression des ersten detektierbaren Reporters durch Folgendes verfeinert wird:
(a) Regulieren der Expression des ersten detektierbaren Reporters unter Verwendung von Promotern mit verschiedenen Stärken; und/oder
(b) Einschließen einer In-Frame-Destabilisierungsnukleotidsequenz in die Nukleotidsequenz des ersten detektierbaren Reporters; und/oder
(c) Einschließen einer Nukleotidsequenz, die Proteine für proteosomalen Abbau anvisiert, in die Nukleotidsequenz, die einen ersten detektierbaren Reporter codiert; und/oder
(d) Ändern der Stabilität des ersten detektierbaren Reporters durch Einschließen von Elementen reich an Adenylat/Uridylat (AREs) in die 3'-untranslatierte Region (UTR) seiner Messenger-RNA,
optional
(4) Identifizieren einer oder mehrerer Zellen in der Population, die den ersten detektierbaren Reporter, aber nicht den zweiten detektierbaren Reporter exprimieren, wobei die in die eine oder mehreren Zellen eingeführte Testverbindung ein putativer Hemmer des Zellsignalisierungsweges ist,
wobei der Zellsignalisierungsweg bevorzugt konstitutiv in der Säugetierzelle aktiviert wird,
wobei die Population an Säugetierzellen bevorzugt bereitgestellt wird, indem eine Population an Säugetierzellen mit der ersten und zweiten heterologen Nukleinsäure transfiziert wird,
wobei die erste und zweite heterologe Nukleinsäure bevorzugt innerhalb des gleichen oder separater Expressionsvektoren enthalten sind,
wobei das Repressormolekül bevorzugt eine miRNA ist.

2. Verfahren nach Anspruch 1, wobei der erste und zweite detektierbare Reporter fluoreszierende Proteine sind, bevorzugt jeweils Cherry und emGFP,
wobei die Expression des ersten und zweiten detektierbaren Reporters bevorzugt durch Messen der fluoreszierenden Emission der Reporter bestimmt wird.

3. Verfahren nach Anspruch 1 oder Anspruch 2, wobei die Testverbindungen Biomoleküle sind, bevorzugt Peptide, bevorzugter Phylomere.

4. Verfahren nach Anspruch 3, wobei die Bibliothek von Testverbindungen in die Population an Säugetierzellen eingeführt wird, indem eine Bibliothek von Nukleinsäuren, die eine unterschiedliche Population an Testbiomolekülen in der Population codiert, exprimiert wird,
bevorzugt umfassend den zusätzlichen Schritt, vor Schritt (2), des Transfizierens der Population an Säugetierzellen mit der Bibliothek von Nukleinsäuren, die die Population an Testbiomolekülen codiert,
wobei die Bibliothek von Nukleinsäuren bevorzugt 1 x 10⁶ oder mehr unterschiedliche Testbiomoleküle codiert,
wobei die Testbiomoleküle bevorzugt Peptide sind, bevorzugter Phylomere.

5. Verfahren nach Anspruch 3 oder Anspruch 4, umfassend das Isolieren einer oder mehrerer Säugetierzellen, die den ersten detektierbaren Reporter, aber nicht den zweiten detektierbaren Reporter exprimieren, wobei die Zellen bevorzugt durch fluoreszenzaktiviertes Zellsortieren isoliert werden.

6. Verfahren nach Anspruch 5, wobei die eine oder mehreren isolierten Zellen kultiviert und/oder expandiert werden.

7. Verfahren nach Anspruch 5 oder Anspruch 6, umfassend das Amplifizieren, Klonen und/oder Sequenzieren der Nukleinsäure, die das Testbiomolekül aus der einen oder den mehreren Säugetierzellen codiert.

8. Verfahren nach einem der Ansprüche 5 bis 7, umfassend das Isolieren von Nukleinsäuren, die Testbiomoleküle aus der einen oder den mehreren Säugetierzellen codieren, die den ersten detektierbaren Reporter, aber nicht den zweiten detektierbaren Reporter exprimieren.

9. Verfahren nach Anspruch 7 oder Anspruch 8, umfassend
(5) Bereitstellen einer weiteren Population an Säugetierzellen, umfassend eine erste und eine zweite heterologe Nukleinsäure gemäß Schritt (1) aus Anspruch 1,
(6) Transfizieren der weiteren Population an Zellen mit der Population an Nukleinsäuren, die Testbiomoleküle codieren, isoliert von der einen oder den mehreren Säugetierzellen, die positiv für den ersten detektierbaren Reporter und negativ für den zweiten detektierbaren Reporter sind,
(7) Exprimieren der Population an Nukleinsäuren in der weiteren Population der Säugetierzellen, und
(8) Bestimmen der Expression des ersten und des zweiten detektierbaren Reporters in den Säugetierzellen,
wobei Expression des ersten detektierbaren Reporters, aber nicht des zweiten detektierbaren Reporters darauf hinweist, dass das Testbiomolekül, das durch eine Nukleinsäure in einer Säugetierzelle exprimiert wird, ein Hemmer des Zellsignalisierungsweges ist,
(9) Identifizieren einer oder mehrerer Säugetierzellen in der weiteren Population, die den ersten detektierbaren Reporter, aber nicht den zweiten detektierbaren Reporter exprimieren, und
(10) Isolieren der einen oder mehreren Säugetierzellen, die den ersten detektierbaren Reporter, aber nicht den zweiten detektierbaren Reporter exprimieren,
optional umfassend das Isolieren von Nukleinsäuren, die Testbiomoleküle aus der einen oder den mehreren Säugetierzellen, die in Schritt (10) isoliert wurden, codieren,
wobei optional Schritt (5) bis (10) ein- oder mehrmals wiederholt werden.

10. Verfahren nach Anspruch 4, wenn die Bibliothek von Nukleinsäuren 1 x 10⁶ oder mehr unterschiedliche Testbiomoleküle codiert, und Anspruch 5 bis 9, umfassend das Exprimieren einer Nukleinsäure isoliert von einer Säugetierzelle, die den ersten detektierbaren Reporter, aber nicht den zweiten detektierbaren Reporter exprimiert, um das Testbiomolekül zu erzeugen.

11. Verfahren zum Identifizieren eines Proteins, einer Proteinregion oder einer Stelle der Protein:Protein-Interaktion (PPI), das/die ein nützliches Ziel für die therapeutische Modulation eines Zellsignalisierungsweges sein kann, wobei das Verfahren Folgendes umfasst:
Screening auf eine Verbindung, die einen Zellsignalisierungsweg hemmt, nach einem der vorhergehenden Ansprüche,
Bereitstellen einer Testverbindung, die bewirkt, dass eine Säugetierzelle den ersten detektierbaren Reporter, aber nicht den zweiten detektierbaren Reporter detektiert, in dem Verfahren nach einem der vorhergehenden Ansprüche,
Identifizieren eines intrazellulären Bindungspartners, der die Testverbindung bindet, die bewirkt, dass eine Säugetierzelle den ersten detektierbaren Reporter, aber nicht den zweiten detektierbaren Reporter exprimiert, in dem Verfahren nach einem der vorhergehenden Ansprüche, wobei der Bindungspartner ein Kandidatenzielprotein zur Modulation eines Zellsignalisierungsweges ist,
wobei bevorzugt die Testverbindung, die in dem Verfahren nach einem der vorhergehenden Ansprüche bewirkt, dass eine Säugetierzelle den ersten detektierbaren Reporter, aber nicht den zweiten detektierbaren Reporter detektiert, ein Biomolekül ist, das durch eine Nukleinsäure von einer Säugetierzelle codiert wird.

12. Verfahren nach Anspruch 11, wenn die Testverbindung, die in dem Verfahren nach einem der vorhergehenden Ansprüche bewirkt, dass eine Säugetierzelle den ersten detektierbaren Reporter, aber nicht den zweiten detektierbaren Reporter exprimiert, ein Biomolekül ist, das durch eine Nukleinsäure von einer Säugetierzelle codiert wird, wobei das Verfahren das Identifizieren einer Region des intrazellulären Bindungspartners umfasst, der an das Testbiomolekül bindet, wobei die Region eine Kandidatenzielregion oder Stelle zur Modulation eines Zellsignalisierungsweges ist.

13. Verfahren nach Anspruch 11 oder Anspruch 12, wobei das Zielprotein, die Proteinregion oder die Stelle der Protein:Protein-Interaktion (PPI) eine phänotypische Reaktion in einer Säugetierzelle moduliert.

14. Kultivierte Säugetierzelle oder Population an Säugetierzellen, wobei jede Zelle Folgendes umfasst:
(a) eine erste heterologe Nukleinsäure, umfassend:
(i) eine Nukleotidsequenz, die einen ersten detektierbaren Reporter codiert, und
(ii) ein konstitutives regulatorisches Element, das mit der Nukleotidsequenz wirkverbunden ist; und
(b) eine zweite heterologe Nukleinsäure, umfassend:
(i) eine Nukleotidsequenz, die ein Repressormolekül codiert, das die Expression des ersten detektierbaren Reporters hemmt, deaktiviert oder unterdrückt,
(ii) eine Nukleotidsequenz, die einen zweiten detektierbaren Reporter codiert, und
(iii) ein signalaktiviertes regulatorisches Element, das durch den Zellsignalisierungsweg aktiviert wird,
wobei das signalaktivierte regulatorische Element mit der Nukleotidsequenz, die das Repressormolekül codiert, und der Nukleotidsequenz, die den zweiten detektierbaren Reporter codiert, wirkverbunden ist,
wobei die Expression des ersten detektierbaren Reporters durch Folgendes verfeinert wird:
(a) Regulieren der Expression des ersten detektierbaren Reporters unter Verwendung von Promotern mit verschiedenen Stärken; und/oder
(b) Einschließen einer In-Frame-Destabilisierungsnukleotidsequenz in die Nukleotidsequenz des ersten detektierbaren Reporters; und/oder
(c) Einschließen einer Nukleotidsequenz, die Proteine für proteosomalen Abbau anvisiert, in die Nukleotidsequenz, die einen ersten detektierbaren Reporter codiert; und/oder
(d) Ändern der Stabilität des ersten detektierbaren Reporters durch Einschließen von Elementen reich an Adenylat/Uridylat (AREs) in die 3'-untranslatierte Region (UTR) seiner Messenger-RNA,
die bevorzugt mit einer Bibliothek von Nukleinsäuren transfiziert ist, die eine unterschiedliche Population an Testbiomolekülen codiert,
wobei das Repressormolekül bevorzugt miRNA ist.

15. Vektor oder Kombination an Vektoren, der/die Folgendes umfasst:
(a) eine erste heterologe Nukleinsäure, umfassend:
(i) eine Nukleotidsequenz, die einen ersten detektierbaren Reporter codiert, und
(ii) ein konstitutives regulatorisches Element, das mit der Nukleotidsequenz wirkverbunden ist; und
(b) eine zweite heterologe Nukleinsäure, umfassend:
(i) eine Nukleotidsequenz, die ein Repressormolekül codiert, das die Expression des ersten detektierbaren Reporters hemmt, deaktiviert oder unterdrückt,
(ii) eine Nukleotidsequenz, die einen zweiten detektierbaren Reporter codiert, und
(iii) ein signalaktiviertes regulatorisches Element, das durch den Zellsignalisierungsweg aktiviert wird,
wobei das signalaktivierte regulatorische Element mit der Nukleotidsequenz, die das Repressormolekül codiert, und der Nukleotidsequenz, die den zweiten detektierbaren Reporter codiert, wirkverbunden ist,
wobei die Expression des ersten detektierbaren Reporters durch Folgendes verfeinert wird:
(a) Regulieren der Expression des ersten detektierbaren Reporters unter Verwendung von Promotern mit verschiedenen Stärken; und/oder
(b) Einschließen einer In-Frame-Destabilisierungsnukleotidsequenz in die Nukleotidsequenz des ersten detektierbaren Reporters; und/oder
(c) Einschließen einer Nukleotidsequenz, die Proteine für proteosomalen Abbau anvisiert, in die Nukleotidsequenz, die einen ersten detektierbaren Reporter codiert; und/oder
(d) Ändern der Stabilität des ersten detektierbaren Reporters durch Einschließen von Elementen reich an Adenylat/Uridylat (AREs) in die 3'-untranslatierte Region (UTR) seiner Messenger-RNA,
wobei das Repressormolekül bevorzugt miRNA ist.

16. Verfahren nach einem der Ansprüche 1 bis 13, Zelle nach Anspruch 14 oder Vektor nach Anspruch 15, wobei die Expression des ersten detektierbaren Reporters durch (b) verfeinert wird, wobei die In-Frame-Destabilisierungssequenz eine PEST-Sequenz ist.

17. Verfahren nach einem der Ansprüche 1 bis 13 oder 16, Zelle nach Anspruch 14 oder Vektor nach Anspruch 15, wobei die Expression des ersten detektierbaren Reporters durch (c) verfeinert wird, wobei die Nukleotidsequenz, die Proteine für proteosomalen Abbau anvisiert, eine CL1-Degron -Sequenz ist.

## Revendications

1. Procédé de criblage pour un composé qui inhibe une voie de signalisation cellulaire comprenant :
(1) la fourniture d'une population de cellules mammaliennes cultivées ayant une voie de signalisation cellulaire active, chaque cellule mammalienne comprenant :
(a) un premier acide nucléique hétérologue comprenant :
(i) une séquence nucléotidique codant pour un premier rapporteur détectable et,
(ii) un élément régulateur constitutif qui est lié de manière fonctionnelle à la séquence nucléotidique ; et,
(b) un deuxième acide nucléique hétérologue comprenant :
(i) une séquence nucléotidique codant pour une molécule de répresseur qui inhibe, inactive ou supprime l'expression du premier rapporteur détectable,
(ii) une séquence nucléotidique codant pour un deuxième rapporteur détectable ; et,
(iii) un élément régulateur activé par un signal qui est activé par ladite voie de signalisation cellulaire,
ledit élément régulateur activé par un signal étant lié de manière fonctionnelle à la séquence nucléotidique de répresseur et à la séquence nucléotidique qui code pour le deuxième rapporteur détectable,
(2) l'introduction d'une banque de composés d'essai dans ladite population de cellules mammaliennes, et ;
(3) la détermination de l'expression des premier et deuxième rapporteurs détectables dans une ou plusieurs cellules de la population de cellules transfectées,
dans lequel l'expression du premier rapporteur détectable mais pas du deuxième rapporteur détectable dans une cellule transfectée est une indication que le composé d'essai inhibe ladite voie de signalisation cellulaire,
dans lequel l'expression du premier rapporteur détectable est affinée par :
(a) la régulation de l'expression du premier rapporteur détectable en utilisant des promoteurs de forces différentes ; et/ou
(b) l'inclusion d'une séquence nucléotidique de déstabilisation interne au cadre dans la séquence nucléotidique du premier rapporteur détectable ; et/ou
(c) l'inclusion d'une séquence nucléotidique qui cible des protéines pour la dégradation protéasomale dans la séquence nucléotidique codant pour un premier rapporteur détectable ; et/ou
(d) l'altération de la stabilité du premier rapporteur détectable en incorporant des éléments riches en adénylate-uridylate (ERA) dans la région non traduite 3' (UTR) de son ARN messager,
facultativement,
(4) l'identification d'une ou de plusieurs cellules dans la population qui expriment le premier rapporteur détectable mais pas le deuxième rapporteur détectable, dans lequel le composé d'essai introduit dans les une ou plusieurs cellules est un inhibiteur putatif de la voie de signalisation cellulaire,
préférablement dans lequel la voie de signalisation cellulaire est activée de manière constitutive dans la cellule mammalienne,
préférablement dans lequel la population de cellules mammaliennes est fournie par transfection d'une population de cellules mammaliennes avec les premier et deuxième acides nucléiques hétérologues, préférablement dans lequel les premier et deuxième acides nucléiques hétérologues sont compris dans les mêmes vecteurs d'expression ou des vecteurs d'expression distincts, préférablement dans lequel la molécule de répresseur est un miARN.

2. Procédé selon la revendication 1 dans lequel les premier et deuxième rapporteurs détectables sont des protéines fluorescentes, préférablement Cherry et emGFP respectivement,
préférablement dans lequel l'expression des premier et deuxième rapporteurs détectables est déterminée en mesurant l'émission fluorescente des rapporteurs.

3. Procédé selon la revendication 1 ou la revendication 2 dans lequel les composés d'essai sont des biomolécules, préférablement des peptides, plus préférablement des phylomères.

4. Procédé selon la revendication 3 dans lequel la banque de composés d'essai est introduite dans la population de cellules mammaliennes par l'expression d'une banque d'acides nucléiques codant pour une population diverse de biomolécules d'essai dans ladite population,
préférablement comprenant l'étape supplémentaire avant l'étape (2) de transfection de la population de cellules mammaliennes avec la banque d'acides nucléiques codant pour la population de biomolécules d'essai,
préférablement dans lequel la banque d'acides nucléiques code pour 1 x 10⁶ ou plus de biomolécules d'essai diverses,
préférablement dans lequel les biomolécules d'essai sont des peptides, plus préférablement des phylomères.

5. Procédé selon la revendication 3 ou la revendication 4 comprenant l'isolement d'une ou de plusieurs cellules mammaliennes qui expriment le premier rapporteur détectable mais pas le deuxième rapporteur détectable, préférablement dans lequel les cellules sont isolées par un tri cellulaire activé par la fluorescence.

6. Procédé selon la revendication 5 dans lequel les une ou plusieurs cellules isolées sont cultivées et/ou développées.

7. Procédé selon la revendication 5 ou la revendication 6 comprenant l'amplification, le clonage et/ou le séquençage de l'acide nucléique codant pour la biomolécule d'essai à partir desdites une ou plusieurs cellules mammaliennes.

8. Procédé selon l'une quelconque des revendications 5 à 7 comprenant l'isolement des acides nucléiques codant pour les biomolécules d'essai à partir des une ou plusieurs cellules mammaliennes qui expriment le premier rapporteur détectable mais pas le deuxième rapporteur détectable.

9. Procédé selon la revendication 7 ou la revendication 8 comprenant
(5) la fourniture d'une population supplémentaire de cellules mammaliennes comprenant un premier et un deuxième acides nucléiques hétérologues selon l'étape (1) de la revendication 1,
(6) la transfection de ladite population supplémentaire de cellules avec ladite population d'acides nucléiques codant pour les biomolécules d'essai isolés à partir des une ou plusieurs cellules mammaliennes positives pour le premier rapporteur détectable et négatives pour le deuxième rapporteur détectable,
(7) l'expression de la population d'acides nucléiques dans la population supplémentaire desdites cellules mammaliennes, et
(8) la détermination de l'expression des premier et deuxième rapporteurs détectables dans les cellules mammaliennes,
dans lequel l'expression du premier rapporteur détectable mais pas du deuxième rapporteur détectable est une indication que la biomolécule d'essai exprimée par un acide nucléique dans une cellule mammalienne est un inhibiteur de ladite voie de signalisation cellulaire,
(9) l'identification d'une ou de plusieurs cellules mammaliennes dans la population supplémentaire qui expriment le premier rapporteur détectable mais pas le deuxième rapporteur détectable, et ;
(10) l'isolement des une ou plusieurs cellules mammaliennes qui expriment le premier rapporteur détectable mais pas le deuxième rapporteur détectable,
comprenant facultativement l'isolement d'acides nucléiques codant pour les biomolécules d'essai à partir des une ou plusieurs cellules mammaliennes isolées dans l'étape (10),
facultativement dans lequel les étapes (5) à (10) sont répétées une ou plusieurs fois.

10. Procédé selon la revendication 4 quand la banque d'acides nucléiques code pour 1 x 10⁶ ou plus de biomolécules d'essai diverses, et les revendications 5 à 9 comprenant l'expression d'un acide nucléique isolé à partir d'une cellule mammalienne qui exprime le premier rapporteur détectable mais pas le deuxième rapporteur détectable pour produire la biomolécule d'essai.

11. Procédé d'identification d'une protéine, d'une région de protéine ou d'un site d'interaction protéine/protéine (IPP) qui pourrait être une cible utile pour la modulation thérapeutique d'une voie de signalisation cellulaire, le procédé comprenant :
le criblage pour un composé qui inhibe une voie de signalisation cellulaire selon l'une quelconque des revendications précédentes,
la fourniture d'un composé d'essai qui amène la cellule mammalienne à exprimer le premier rapporteur détectable mais pas le deuxième rapporteur détectable dans le procédé selon l'une quelconque des revendications précédentes,
l'identification d'un partenaire de liaison intracellulaire qui se lie au composé d'essai qui amène une cellule mammalienne à exprimer le premier rapporteur détectable mais pas le deuxième rapporteur détectable dans le procédé selon l'une quelconque des revendications précédentes, ledit partenaire de liaison étant une protéine cible candidate pour la modulation d'une voie de signalisation cellulaire,
préférablement dans lequel le composé d'essai qui amène une cellule mammalienne à exprimer le premier rapporteur détectable mais pas le deuxième rapporteur détectable dans le procédé selon l'une quelconque des revendications précédentes est une biomolécule qui est codée par un acide nucléique à partir d'une cellule mammalienne.

12. Procédé selon la revendication 11 quand le composé d'essai qui amène une cellule mammalienne à exprimer le premier rapporteur détectable mais pas le deuxième rapporteur détectable dans le procédé selon l'une quelconque des revendications précédentes est une biomolécule qui est codée par un acide nucléique à partir d'une cellule mammalienne, le procédé comprenant l'identification d'une région du partenaire de liaison intracellulaire qui se lie à la biomolécule d'essai, ladite région étant une région cible candidate ou un site pour la modulation d'une voie de signalisation cellulaire.

13. Procédé selon la revendication 11 ou la revendication 12 dans lequel la protéine cible, la région de protéine ou le site d'interaction protéine/protéine (IPP) module une réponse phénotypique dans une cellule mammalienne.

14. Cellule mammalienne cultivée ou une population de cellules mammaliennes, chaque cellule comprenant :
(a) un premier acide nucléique hétérologue comprenant ;
(i) une séquence nucléotidique codant pour un premier rapporteur détectable et,
(ii) un élément régulateur constitutif qui est lié de manière fonctionnelle à la séquence nucléotidique ; et,
(b) un deuxième acide nucléique hétérologue comprenant :
(i) une séquence nucléotidique codant pour une molécule de répresseur qui inhibe, inactive ou supprime l'expression du premier rapporteur détectable,
(ii) une séquence nucléotidique codant pour un deuxième rapporteur détectable ; et,
(iii) un élément régulateur activé par un signal qui est activé par ladite voie de signalisation cellulaire,
ledit élément régulateur activé par un signal étant lié de manière fonctionnelle à la séquence nucléotidique qui code pour la molécule de répresseur et à la séquence nucléotidique qui code pour le deuxième rapporteur détectable,
dans lequel l'expression du premier rapporteur détectable est affinée par :
(a) la régulation de l'expression du premier rapporteur détectable en utilisant des promoteurs de forces différentes ; et/ou
(b) l'inclusion d'une séquence nucléotidique de déstabilisation interne au cadre dans la séquence nucléotidique du premier rapporteur détectable ; et/ou
(c) l'inclusion d'une séquence nucléotidique qui cible des protéines pour la dégradation protéasomale dans la séquence nucléotidique codant pour un premier rapporteur détectable ; et/ou
(d) l'altération de la stabilité du premier rapporteur détectable en incorporant des éléments riches en adénylate-uridylate (ERA) dans la région non traduite 3' (UTR) de son ARN messager, facultativement,
préférablement qui est transfectée avec une banque d'acides nucléiques codant pour une population diverse de biomolécules d'essai,
préférablement dans lequel la molécule de répresseur est un miARN.

15. Vecteur ou combinaison de vecteurs qui comprend :
(a) un premier acide nucléique hétérologue comprenant ;
(i) une séquence nucléotidique codant pour un premier rapporteur détectable et,
(ii) un élément régulateur constitutif qui est lié de manière fonctionnelle à la séquence nucléotidique ; et,
(b) un deuxième acide nucléique hétérologue comprenant :
(i) une séquence nucléotidique codant pour une molécule de répresseur qui inhibe, inactive ou supprime l'expression du premier rapporteur détectable,
(ii) une séquence nucléotidique codant pour a deuxième rapporteur détectable ; et,
(iii) un élément régulateur activé par un signal qui est activé par ladite voie de signalisation cellulaire,
ledit élément régulateur activé par un signal étant lié de manière fonctionnelle à la séquence nucléotidique qui code pour la molécule de répresseur et à la séquence nucléotidique qui code pour le deuxième rapporteur détectable,
dans lequel l'expression du premier rapporteur détectable est affinée par :
(a) la régulation de l'expression du premier rapporteur détectable en utilisant des promoteurs de forces différentes ; et/ou
(b) l'inclusion d'une séquence nucléotidique de déstabilisation interne au cadre dans la séquence nucléotidique du premier rapporteur détectable ; et/ou
(c) l'inclusion d'une séquence nucléotidique qui cible des protéines pour la dégradation protéasomale dans la séquence nucléotidique codant pour un premier rapporteur détectable ; et/ou
(d) l'altération de la stabilité du premier rapporteur détectable en incorporant des éléments riches en adénylate-uridylate (ERA) dans la région non traduite 3' (UTR) de son ARN messager, facultativement,
préférablement dans lequel la molécule de répresseur est un miARN.

16. Procédé selon l'une quelconque des revendications 1 à 13, cellule selon la revendication 14 ou vecteur selon la revendication 15 dans lequel l'expression du premier rapporteur détectable est ajustée par (b), dans lequel la séquence de déstabilisation interne au cadre est une séquence PEST.

17. Procédé selon l'une quelconque des revendications 1 à 13 ou 16, cellule selon la revendication 14 ou vecteur selon la revendication 15 dans lequel l'expression du premier rapporteur détectable est ajustée par (c), dans lequel la séquence nucléotidique qui cible des protéines pour la dégradation protéasomale est une séquence degron CL1.


### SEQUENCE LISTING

<110> Cambridge Enterprise Limited
<120> A Method of Screening for Modulation of Cell Signalling Pathways
<150> GB1420218.8
   <151> 2014-11-13
<160> 2
<170> BiSSAP 1.2
<210> 1
   <211> 59
   <212> RNA
   <213> Artificial Sequence
<220>
   <221> source
   <222> 1..59
   <223> /organism="Artificial Sequence" /note="Seq ID 1" /mol_type="unassigned DNA"
<220>
   <221> misc_feature
   <222> 12,13
   <223> /note="modified"
<400> 1
   ttgatgttga cgttgtaggc ggttttggcc actgactgac cgcctacagt caacatcaa 59
<210> 2
   <211> 10
   <212> DNA
   <213> Artificial Sequence
<220>
   <221> source
   <222> 1..10
   <223> /organism="Artificial Sequence" /note="Wnt" /mol_type="unassigned DNA"
<400> 2
   agatcaaagg 10